# EUROPEAN PATENT SPECIFICATION

(11) **EP 1 317 233 B1**
(45) Date of publication and mention of the grant of the patent: **27.06.2007**
(21) Application number: 01965806.1
(22) Date of filing: 12.09.2001
(51) Int. Cl.: A61F 13/64

(54) **ABSORBENT ARTICLE WITH ADJUSTABLE ATTACHMENT OF FASTENING MEANS**
ABSORBIERENDER ARTIKEL MIT EINSTELLBAREM BEFESTIGUNGSMITTEL
ARTICLE ABSORBANT A FERMETURE REGLABLE

(30) Priority: 12.09.2000 SE 0003231
(43) Date of publication of application: 11.06.2003
(73) Proprietor: SCA Hygiene Products AB, 405 03 Göteborg (SE)
(72) Inventor: HERMANSSON, Kent, S-426 54 Västra Frölunda (SE); RAGNARSSON, Christina, S-412 69 Göteborg (SE); JOHANSSON, Asa, S-417 16 Göteborg (SE)
(74) Representative: Egeröd, Lisbeth
(86) International application number: PCT/SE2001/001949
(87) International publication number: WO 2002/022064

(56) References cited:
- GB-A- 2 267 024
- US-A- 3 618 608
- US-A- 5 368 585
- US-A- 5 445 628

## Description

### Technical field

The present invention refers to an absorbent article such as a diaper and an incontinence guard comprising a liquid permeable topsheet, a liquid impermeable backsheet and an absorbent body enclosed therebetween, said article having a front portion, a rear portion and a crotch portion therebetween, and further is provided with a pair of belt portions, said belt portions being permanently integrated with the rear portion alternatively the front portion of the article and which are intended to be fastened together around the waist of the wearer and where said front portion alternatively the rear portion is provided with attachment means intended to be attached to the belt portions, in such a way that the article will assume a pantlike shape, where the belt portions form a part of the waist portions of the pant.

### Background of the invention

Diapers and incontinence guards for incontinent adults usually have a garment portion holding an absorbent body in place against the user's body and attachment means which hold the garment portion in place also when the user is moving. A common type of attachment means are adhesive tapes or hook and loop fasteners of the touch-and-close type which directly attach the front and rear portions of the absorbent article to each other.
It is further known, through e.g., EP-A-0 287 388, EP-A-0 409 307, EP-A-0 528 282, EP-A-0 605 012 and FR-A-2 586 558, to attach the front and rear portions of the article by means of a belt, at which the possibilities to adjust the fit are improved. The belt further provides a simplified change of diaper or incontinence guard, especially when the patient is standing up.

On a common type of belt diaper the belt portions are first attached around the waist on the patient and then the front portion of the diaper is attached to the outside of the belt using hook and loop fasteners, being arranged on the belt and the front portion respectively. One problem with diapers provided with hook and loop fasteners and a plastic backsheet material, is that on smaller persons the tab having the hook and loop fasteners on the front portion reaches all the way to the plastic backsheet on the rear portion of the diaper. The hook and loop fasteners can not attach to this plastic material. If in this case the front portion is anyhow attached to the belt, this will lead to a poor fit of the diaper.

Document WO 97/31605 discloses a diaper having extended side portions, exhibiting fastening means at the ends intended to be attached against corresponding fastening means arranged at the front portion of the diaper. A number of fastening means are arranged on the front portion for an adaptation to different waist sizes. However, the diaper shown in the document is not a belt diaper in accordance with the present invention, where the belt portions are attached around the waist of the wearer, whereafter the front portion of the diaper is attached against the belt portions. Therefore, it does not solve the problem with an increased fit for belt diapers and in particular diapers exhibiting hook and loop fasteners.

### Summary of the invention

The object of the present invention is to accomplish a belt-provided absorbent article, such as a diaper or incontinence guard according to the above given definition, exhibiting an improved fit. This object is being solved in that the diaper comprises at least one second fastening means being of the hook and loop fasteners type, being arranged inside said first fastening means on the front portion of the article. This second fastening means attach to the belt portions, which leads to a good fit of the diaper also on a person having a smaller waist size.

### Short description of the drawings

The invention will in the following be closer described with reference to an embodiment shown in the accompanying drawings.

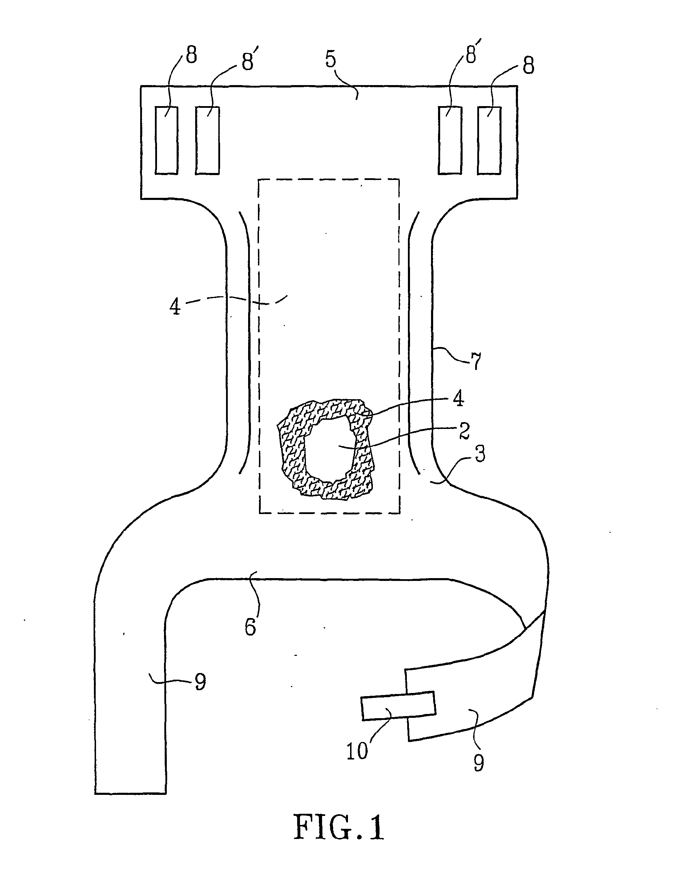

Fig. 1 shows schematically a perspective view of a diaper or incontinence guard according to the invention.

### Detailed description of the invention

The drawing shows an embodiment of a diaper or incontinence guard 1 comprising a liquid impermeable backsheet 2, a liquid permeable topsheet 3 and an absorbent body 4 enclosed therebetween. The liquid permeable topsheet 3 can consist of a nonwoven material, e.g., a spunbond material of continuous filaments, a meltblown material, a bonded carded fibrous web or a perforated plastic film. The liquid impermeable backsheet 2 may consist of a plastic film, a nonwoven material coated with a liquid impervious material or a hydrophobic nonwoven material which resists liquid penetration. In the present invention the backsheet material consists of a plastic film, which can not function as a reception surface for hook and loop fasteners.

The topsheet 3 and the backsheet material 2 has a somewhat greater extension in the plane than the absorbent body 4 and extends outside the edges thereof. The layers 2 and 3 are connected to each other within the projecting portions thereof, e.g., by gluing or welding by heat or ultrasonic.

The absorbent body 4 can be of any conventional kind. Examples of commonly occurring absorbent materials are cellulosic fluff pulp, tissue layers, highly absorbent polymers (so called superabsorbents), absorbent foam materials, absorbent nonwovens or the like. It is common to combine cellulosic fluff pulp with superabsorbents in an absorbent body. It is also common to have absorbent bodies comprising layers of different material with different properties with respect to liquid acquisition capacity, liquid distribution capacity and storage capacity. It is well-known to the person skilled in the art and does therefore not have to be described in detail. The thin absorbent bodies which are common in for example baby diapers and incontinence guards often comprise a compressed mixed or layered structure of cellulosic fluff pulp and superabsorbent.

The diaper/incontinence guard is intended to enclose the lower part of the wearer's trunk like a pair of absorbent pants. It comprises a front portion 5 intended during use to be worn on the front part of the user's body, a rear portion 6 intended during use to be worn on the rear part of the user's body, and a more narrow crotch portion 7 located between the front and rear portions and which is intended to be worn in the crotch part of the user between the legs.

A pair of belt portions 9 are with one end attached, e. g., glued or ultrasonically welded, to the rear portion 6 of the diaper. The belt portions 9 are with their opposite ends intended to be fastened together, e. g. by means of fastening means 10 which is attached against the outside of the opposite belt portion. The fastening means may be a hook-and-loop type fasteners or tape tabs. The attachment tapes 8 of the front portion 5 comprises one part of a hook-and-loop type fastener and is provided with a surface having hook-material. The outside portions of the belt portions 9 comprises the reception surface for the hook-material. This reception surface, the so-called loop portion is preferably comprised of a non-woven material. Said fastening means 8 is intended to be attached against the outsides of the belt portions 9 in order to fasten together the diaper/incontinence guard to the desired pantlike shape.

According to an alternative embodiment the belt portions are attached to the front portion 5 of the diaper and thus are intended to be fastened together on the back of the wearer. The fastening means 8 are then arranged on the rear portion 6 of the diaper.

The width of the belt portions 9 should be between 5-20 cm, preferably between 7-15 cm.

The belt portions 9 are preferably a laminate of a carrier material, which forms the outside of the belt, and a soft nonwoven, which forms the inside of the belt intended to be in direct contact with the skin of the user. A suitable nonwoven material can be a spunbond material of e.g. polypropylene- or polyethylene fibres. Conjugate fibres may also be used. Another suitable nonwoven material can be a carded thermobonded material of e.g., polypropylene- , polyester- or conjugate fibres. Also elastic laminates are suitable to use as material in the belt portions. The carrier material should be adapted to function as a reception surface for both the attachment means 8 and 10, wherein in those cases the attachment means are hook-and-loop type fasteners, nonwoven materials are suitable.

Said fastening means 8 is comprised of surfaces having hook-material. The hook-material surface, i.e. the fastening means 8, is arranged on a suitable location at the front portion for attachment to the belt. Inside this first fastening means 8 a second fastening means 8' is arranged on a suitable distance. Fastening means 8 and 8' may also be arranged on a tab which can be arranged in a way so that it projects a certain distance from the edge of the front portion 5. When the product is applied on a normally sized person, both fastening means is used for attachment against the belt. However, on a smaller person, where the fastening means 8 extend beyond the belt portions 9 all the way to the backsheet material 2, only the second fastening means 8' is used for attachment to the belt, since the first fastening means 8 is not capable to attach against the plastic material on the rear portion of the product. The front portion may also comprise a number of secondary fastening means 8'.

The diaper may now be adapted to different persons depending on their size with remained fit. It leads to a higher flexibility on e.g. hospitals and homes for elderly people, when the same diaper size may be used to an increased number of persons.

The invention is of course not limited to the above described embodiment but can be modified within the scope of the claim.

## Claims

1. Absorbent article such as a diaper and an incontinence guard comprising a liquid permeable topsheet (3), a liquid impermeable backsheet (2) and an absorbent body (4) enclosed therebetween, said article having a front portion (5), a rear portion (6) and a crotch portion (7) therebetween, and further is provided with a pair of belt portions (9), said belt portions (9) being permanently integrated with the rear portion (6) alternatively the front portion (5) of the article and which are intended to be fastened together around the waist of the wearer and wherein the other of said front portion (5) and the rear portion (6) includes portions of a first hook and loop fastener (8) which are adapted to attach the other of the front portion (5) and the rear portion (6) to the outside surface of the belt portions (9), in such a way that the article will assume a pantlike shape, wherein the belt portions (9) form a part of the waist portion of the pant,
**characterized in,**
**that** at least one second hook and loop fastener element (8') is arranged inside the portions of the first hook and loop fastener (8) on the other of the front portion (5) and the rear portion (6) of the article, and there is a space between the portions of the first hook and loop fastener (8) and the second hook and loop fastener element (8'), and the space does not include any hook and loop material and the second hook and loop fastener element (8') is adapted to an outside surface of the belt portion (9).

2. The absorbent article according claim 1, wherein the respective portions of the first hook and loop fastener (8) included on the other of the front portion (5) and the rear portion (6) comprise two separate fastener elements and the second hook and loop fastener elements (8') is arranged between the two separate fastener elements (8).

3. The absorbent article according claim 1, wherein there are two second hook and loop fastener elements (8') arranged inside the first hook and loop fastener (8) on the other of the front portion (5) and the rear portion (6).

4. The absorbent article according claim 2, wherein there are two second hook and loop fastener elements (8') is arranged between the two separate fastener elements (8).

5. The absorbent article according claim 4, wherein the two separate fastener elements (8) and the two second hook and loop fastener elements (8') is arranged in a single row on the other of the front portion (5) and the rear portion (6).

## Patentansprüche

1. Absorbierender Gegenstand wie beispielsweise eine Windel und ein Inkontinenzschutz, umfassend eine flüssigkeitsdurchlässige Oberlage (3), eine flüssigkeitsundurchlässige Decklage (2) und einen dazwischen eingeschlossenen Absorptionskörper (4), wobei der Gegenstand einen Vorderabschnitt (5), einen Hinterabschnitt (6) und einen Schrittabschnitt (7) dazwischen aufweist und ferner mit zwei Gürtelabschnitten (9) versehen ist, die mit dem Hinterabschnitt (6) alternativ dem Vorderabschnitt (5) des Gegenstandes dauerhaft integriert sind und dazu gedacht sind um die Taille des Trägers befestigt zu werden und wobei der andere des Vorderabschnitts (5) und des Hinterabschnitts (6) Abschnitte eines ersten Haken- und Schlaufenbefestigers (8) umfasst, die dazu geeignet sind den anderen des Vorderabschnitts (5) und des Hinterabschnitts (6) derart an der Außenfläche der Gürtelabschnitte (9) anzubringen, dass der Gegenstand eine Höschen ähnliche Form annehmen wird, wobei die Gürtelabschnitte (9) einen Teil des Taillenabschnitts des Höschens bilden,
**dadurch gekennzeichnet, dass** wenigstens ein zweites Haken- und Schlaufenbefestigerelement (8') innerhalb der Abschnitte des ersten Haken- und Schlaufenbefestigers (8) auf dem anderen des Vorderabschnitts (5) und des Hinterabschnitts (6) des Gegenstandes angeordnet ist und ein Zwischenraum zwischen den Abschnitten des ersten Haken- und Schlaufenbefestigers (8) und dem zweiten Haken und Schlaufenbefestigerelement (8') besteht, der kein Haken- und Schlaufenmaterial aufweist und dass das zweite Haken- und Schlaufenbefestigerelement (8') an eine Außenfläche des Gürtelabschnitts (9) angepasst ist.

2. Absorbierender Gegenstand nach Anspruch 1, bei dem entsprechenden Abschnitte des ersten Haken- und Schlaufenbefestigers (8), die auf dem anderen des Vorderabschnitts (5) und des Hinterabschnitts (6) vorgesehen sind, zwei separate Befestigungselemente umfassen und bei dem die zweiten Haken- und Schlaufenbefestigerelemente (8') zwischen den zwei separaten Befestigungselementen (8) angeordnet sind.

3. Absorbierender Gegenstand nach Anspruch 1, bei dem zwei zweite Haken- und Schlaufenbefestigerelemente (8') innerhalb des ersten Haken- und Schlaufenbefestigers (8) auf dem anderen des Vorderabschnitts (5) und des Hinterabschnitts (6) angeordnet sind.

4. Absorbierender Gegenstand nach Anspruch 2, bei dem zwei zweite Haken und Schlaufenbefestiger (8') zwischen den zwei separaten Befestigungselementen (8) angeordnet sind.

5. Absorbierender Gegenstand nach Anspruch 4 bei dem die zwei separaten Befestigungselemente (8) und die zwei zweiten Haken- und Schlaufenbefestigerelemente (8') in einer einzelnen Reihe auf dem anderen des Vorderabschnitts (5) und des Hinterabschnitts (6) angeordnet sind.

## Revendications

1. Article absorbant tel qu'une couche-culotte et une protection contre l'incontinence comprenant une feuille supérieure (3) perméable aux liquides, une feuille arrière (2) imperméable aux liquides et un corps absorbant (4) enfermé entre elles, ledit article ayant une partie avant (5), une partie arrière (6) et une partie entrejambe (7) entre elles, et est en outre pourvu d'une paire de parties de ceinture (9), lesdites parties de ceinture (9) étant intégrées de façon permanente à la partie arrière (6), en variante la partie avant (5) de l'article et qui sont destinées à être attachées ensemble autour de la taille du porteur et dans lequel l'autre desdites partie avant (5) et partie arrière (6) comprend des parties d'une première fermeture à crochets et à boucles (8) qui sont aptes à attacher l'autre des partie avant (5) et partie arrière (6) sur la surface extérieure des parties de ceinture (9), de telle manière que l'article va prendre une forme de culotte, dans laquelle les parties de ceinture (9) forment une partie de la partie taille de la culotte,
**caractérisé en ce qu'**au moins un deuxième élément de fermeture à crochets et à boucles (8') est placé à l'intérieur des parties de la première fermeture à crochets et à boucles (8) sur l'autre des partie avant (5) et partie arrière (6) de l'article, et il y a un espace entre les parties de la première fermeture à crochets et à boucles (8) et le deuxième élément de fermeture à crochets et à boucles (8'), et cet espace ne comporte aucun matériau de crochets et boucles et le deuxième élément de fermeture à crochets et à boucles (8') est adapté à une surface extérieure de la partie de ceinture (9).

2. Article absorbant selon la revendication 1, dans lequel les parties respectives de la première fermeture à crochets et à boucles (8) comprises sur l'autre des partie avant (5) et partie arrière (6) comprennent deux éléments de fermeture distincts et le deuxième élément de fermeture à crochets et à boucles (8') est placé entre les deux éléments de fermeture distincts (8).

3. Article absorbant selon la revendication 1, dans lequel il y a deux deuxièmes éléments de fermeture à crochets et à boucles (8') placés à l'intérieur de la première fermeture à crochets et à boucles (8) sur l'autre des partie avant (5) et partie arrière (6).

4. Article absorbant selon la revendication 2, dans lequel il y a deux deuxièmes éléments de fermeture à crochets et à boucles (8') placés entre les deux éléments de fermeture distincts (8).

5. Article absorbant selon la revendication 4, dans lequel les deux éléments de fermeture distincts (8) et les deux deuxièmes éléments de fermeture à crochets et à boucles (8') sont agencés en une seule rangée sur l'autre des partie avant (5) et partie arrière (6).
